# EUROPEAN PATENT APPLICATION

(11) **EP 4 516 928 A1**
(43) Date of publication of application: **05.03.2025**
(21) Application number: 23796652.8
(22) Date of filing: 07.04.2023
(51) Int. Cl.: C12Q 1/6886

(54) **COMPOSITION FOR DETERMINING WHETHER NUCLEIC ACID IS METHYLATED AND METHOD FOR DETERMINING WHETHER NUCLEIC ACID IS METHYLATED**

(30) Priority: 26.04.2022 KR 20220051454
(71) Applicant: Lepidyne Co., Ltd., Seongdong-gu Seoul 04779 (KR)
(72) Inventor: KIM, Young-Joon, Seoul 06024 (KR); KIM, Si-Cho, Seoul 01446 (KR); HA, Jeong-Sil, Seoul 06024 (KR)
(74) Representative: Grünecker Patent- und Rechtsanwälte PartG mbB
(86) International application number: PCT/KR2023/004709
(87) International publication number: WO 2023/211003

(57) **Abstract**

The present application relates a composition for determining whether a nucleic acid is methylated and a method for determining whether a nucleic acid is methylated.

## Description

### [TECHNICAL FIELD]

The present application relates to a composition for determining whether a nucleic acid is methylated and a method for determining whether a nucleic acid is methylated.

### [BACKGROUND ART]

As a method for measuring the methylation level of a DNA sample, there are qMSP, MethyLight, and MS-HRM, and the like, and all the methods are constructed to relatively confirm the methylation level of each sample. For example, qMSP method determines the methylation level by using a primer which binds only to a methylated (or unmethylated) template for the same amount of DNA sample and measuring a PCR Cq value for a DNA amount produced by amplifying DNA under the corresponding condition in the sample. The Methylight method increases the specificity of the measurement by adding a probe to qMSP to make DNA which specific to a marker to be determined among amplified DNA to bind among the amplified DNA. In addition, the MS-HRM method uses a primer capable of binding to a template strain regardless of a methylation state of a DNA sample, and confirms a signal of difference in melting points of amplified products according to the temperature increase by comprising a certain number of CpGs in an area except for primer binding regions of amplified products.

qMSP and MethyLight analysis has little limitation in primer design and are advantageous of being simple in experimental design, and can implement relatively high sensitivity. However, an additional control marker analysis is required to correct the amount of a sample used for analysis, and the measurement value is corrected using the result, quantitative analysis is indirect and limitative, and the frequency of type 2 error is high. In addition, biased amplification of a specific template has a disadvantage of lowering specificity as it may increase the frequency of non-specific amplification of each DNA sample. In case of MethyLight developed as a way to supplement specificity, a fluorescent probe capable of specifically binding to an amplified product is further required, and this becomes a disadvantage which increases cost and increases difficulty in analysis test design. Moreover, in many cases, specific binding of the probe has a limitation that analysis of a very small amount of a sample is difficult as it reduces amplification efficiency of PCR.

In case of the MS-HRM analysis, as methylation and unmethylation nucleic acids are simultaneously amplified in the same test tube, the methylation level of a DNA sample can be quantitatively measured more intuitively than MSP without additional control marker analysis. However, because of a design limitation that CpG should not be comprised in a primer or a non-specific base should be comprised for primer design, it is not easy to develop a specific analysis method. In addition, in case that CpG is not comprised in a primer, there is a problem of biased binding to an unmethylated strand than a methylated strand, and due to unmethylation bias of the verification result for the sample, type 1 error may occur, and in an experiment requiring sensitive methylation aberration measurement such as cancer diagnosis, there is a problem in that the methylation ratio in an actual sample is measured low or an inaccurate result is derived.

### [DISCLOSURE]

### [TECHNICAL PROBLEM]

An embodiment of the present application is to establish a method for determining methylation status that supplements disadvantages of conventional experimental verification methods. The method for determining methylation status according to an embodiment of the present application can measure a relative methylation ratio without analysis of a control marker in the same test tube, without using a probe which interferes with amplification efficiency, and has high detection sensitivity. In addition, in order to measure the methylation level at a very low ratio in a biological sample such as blood and the like, the amplification efficiency of methylated and unmethylated DNA may be adjusted.

The composition and method according to an embodiment of the present application uses a methylation primer and an unmethylation primer together so it does not generate non-specific amplified products, and can measure the methylation level of a sample more sensitively than a conventional PCR-based analysis method even under a condition in which a desired target nucleic acid is present in a trace amount.

An embodiment of the present application is to provide a composition for determining whether a nucleic acid to be determined is methylated and a method for determining whether a nucleic acid to be determined is methylated with high sensitivity by supplementing disadvantages of conventional methods of measuring the methylation level of a DNA sample.

Another embodiment of the present application is to provide a design strategy and an optimization method of a primer which can be universally applied to various DNA methylation markers.

Other embodiment of the present application is to provide a method for diagnosing cancer, a method for providing information for diagnosing cancer, a composition for diagnosing cancer, or a kit for diagnosing cancer, which determine a DNA methylation level of ctDNA (circulating tumor DNA) present in small amounts in cancer patients among cfDNA (cell-free DNA) present in a biological sample such as blood, through a semi-quantification method in the sample, and based on this, achieve a high sensitivity and specificity.

### [TECHNICAL SOLUTION]

An embodiment of the present application relates to a composition for determining whether a nucleic acid to be determined is methylated, comprising a primer set for amplifying a target site of a nucleic acid to be determined.

Another embodiment of the present application relates to a method for determining whether a nucleic acid to be determined is methylated, comprising a step of modifying a methylated nucleic acid to be determined and an unmethylated nucleic acid to be determined differently from each other, in a nucleic acid to be determined comprised in a biological sample; and a step of amplifying a target site by treating the biological sample with the composition.

Hereinafter, the present application will be described in more detail.

An embodiment of the present application relates to a novel method for measuring the methylation level of a nucleic acid to be determined, which supplements disadvantages of conventional experimental verification methods for measuring the methylation level of a nucleic acid to be determined. Specifically, when designing MS-HRM primers, a methylation primer comprising a CpG recognition site which recognizes a CpG sequence in a primer was used, and to prevent amplifying only a methylated template, an unmethylation primer comprising a TpG recognition site which recognizes a TpG sequence to be used for amplification of an unmethylated template was used together. Then, in order to prevent relatively low sensitivity of MS-HRM and biased amplification of the primer comprising TpG, a method for increasing a binding opportunity of the primer comprising CpG and reducing a binding opportunity of the primer comprising TpG by adjusting the temperature of a primer annealing step of PCR was constructed.

In the present description, the term "methylation" means that a methyl group is attached to a base that compose DNA. For example, methylation may occur in cytosine at a specific CpG site of a specific gene or nucleic acid.

In the present description, the term "whether methylated" or "methylated state" means whether methylated in cytosine at a specific CpG site of a specific gene or nucleic acid. Specifically, it means presence or absence of 5-methyl-cytosine of at least one CpG dinucleotide in a base sequence.

In the present description, the term "methylation level" or "methylation degree" means an amount of methylation present in a base sequence in a nucleic acid to be determined.

In the present description, the term "CpG site" or "CpG sequence" means a CpG site present in a base sequence of a specific gene or nucleic acid. The gene is a concept including all of a series of constituent units which are required for expression and are operably linked to each other, and may comprise a promoter region, a protein coding region (open reading frame, ORF) and a terminator region. Therefore, the CpG site may be present in a promoter region, a protein coding region (open reading frame, ORF) or a terminator region, or the like of the corresponding gene. For example, it may be a CpG site present in a promoter region of the gene.

In the present description, the term "nucleic acid" is a nucleotide in a polymer form, and represents a ribonucleotide or deoxyribonucleotide, and includes meanings such as polynucleotides, oligonucleotides, oligomers, oligos, coding sequences and the like. The nucleic acid may be used as a meaning including single-, double- or multiple-strand DNA or RNA, genome DNA, cDNA, a DNA-RNA hybrid, or purine and pyrimidine bases, or polymers having other naturally, chemically or biochemically modified, non-natural or derivatized nucleotide base.

In the present description, when describing "comprising" a component, it does not mean that other components are exclude but that other components may be further comprised, unless specifically mentioned otherwise.

In the present description, unless otherwise indicated, the nucleic acid is written from left to right, in a direction of 5' to 3', respectively.

In the present description, the singular includes plural objects unless the context clearly indicates otherwise.

One embodiment of the present application relates to a composition for determining whether a nucleic acid to be determined is methylated, comprising a primer set for amplifying a target site of a nucleic acid to be determined, or a kit for determining whether a nucleic acid to be determined is methylated comprising the composition. The primer set may comprise a forward primer and a reverse primer, and one of the forward primer and the reverse primer may comprise a methylation primer and an unmethylation primer, and the other one may be a common primer. The methylation primer may comprise a CpG recognition site which recognizes a CpG sequence of the nucleic acid to be determined, and the unmethylation primer may comprise a TpG recognition site which recognizes a TpG sequence of the nucleic acid to be determined, and the TpG sequence may be converted from the CpG sequence of the nucleic acid to be determined. The common primer means a primer capable of specifically recognizing and binding to a nucleic acid to be determined regardless of whether a nucleic acid to be determined is methylated.

Other embodiment of the present application relates to a method for determining whether a nucleic acid to be determined is methylated, comprising a step of modifying a methylated nucleic acid to be determined and an unmethylated nucleic acid to be determined differently from each other, wherein the nucleic acid to be determined is comprised in a biological sample; and a step of amplifying a target site by treating the biological sample with the composition. The step of modifying may be performed by treating the biological sample with an agent which modifies a methylated nucleic acid to be determined and an unmethylated nucleic acid to be determined differently from each other.

The target site is a site of interest to determine whether a nucleic acid to be determined is methylated, and may comprise at least one CpG sequence. The size of the target site may be 50 to 150 bp, 50 to 140 bp, 50 to 130 bp, 50 to 120 bp, 50 to 110 bp, 50 to 100 bp, 60 to 150 bp, 60 to 140 bp, 60 to 130 bp, 60 to 120 bp, 60 to 110 bp, or 60 to 100 bp.

For example, the target site may comprise a biomarker. Specifically, the biomarker may be a methylation biomarker. The methylation biomarker means a biomarker which shows an association with a specific disease depending on whether the biomarker is methylated. More specifically, the methylation biomarker means a biomarker which shows association with a specific disease depending on an epigenetic change occurring as a methyl group (CH₃) is added to C (cytosine) of CG in the base sequence of the biomarker.

**In** an embodiment of the present application, the target site may comprise a biomarker present in a CpG island. The primer set according to an embodiment of the present application comprises (1) a methylation forward primer, an unmethylation forward primer, and a common reverse primer; or (2) a common forward primer, a methylation reverse primer, and an unmethylation reverse primer, and can accurately determine whether a target site is methylated even when it is difficult to prepare a common primer, for example when sequence complexity is reduced by treatment of sulfurous acid, or when a biomarker is present in a CpG island where changes occur together at a neighboring site. Specifically, in the conventional HRM analysis method, analysis is difficult when the sequence complexity of gDNA is reduced by treatment of sulfurous acid and a non-specific reaction is frequently occur, or in particular, in case of the CpG island, when it is difficult to prepare MIP (Methylation Independent Primer; common primer) not comprising CpG, but the present application can secure the degree of freedom for primer design by using a methylation primer and an unmethylation primer together in a primer of one direction, and can accurately determine whether a target site is methylated while inducing specific binding of a target.

The methylation primer, specifically, the methylation forward primer or the methylation reverse primer comprises a CpG recognition site which recognizes a CpG sequence of the nucleic acid to be determined, and the unmethylation primer, specifically, the unmethylation forward primer or the unmethylation reverse primer comprises a TpG recognition site which recognizes a TpG sequence in which a CpG sequence of the nucleic acid to be determined is converted. The TpG sequence is that a CpG sequence is converted with an agent which modifies a methylated nucleic acid to be determined and an unmethylated nucleic acid to be determined differently from each other. For example, the modifying agent may convert an unmethylated cytosine residue into thymine. As one example, the modifying agent may be at least one selected from the group consisting of sulfurous acid, bisulfite, hydrogen sulfite, and disulfite.

When a template is treated with an agent which modifies a methylated nucleic acid to be determined and an unmethylated nucleic acid to be determined differently from each other, CpG cytosine will not be converted to thymine if CpG cytosine of the template is methylated, and CpG cytosine will be converted to thymine if CpG cytosine is unmethylated.

The CpG recognition site of the methylation primer may recognize a CpG sequence of a methylated nucleic acid to be determined. For example, the CpG recognition site may comprise a CG sequence.

The TpG recognition site of the unmethylation primer may recognize a TpG sequence that a CpG sequence of an unmethylated nucleic acid to be determined is converted by an agent which modifies a methylated nucleic acid to be determined and an unmethylated nucleic acid to be determined differently from each other. For example, the TpG recognition site may comprise a TG sequence.

The CpG recognition site and the TpG recognition site are located near the 3' end of a methylation primer and an unmethylation primer, respectively, and may specifically recognize a methylated nucleic acid to be determined and an unmethylated nucleic acid to be determined which are converted by an agent which modifies a methylated nucleic acid to be determined and an unmethylated nucleic acid to be determined differently from each other, respectively. For example, the CpG recognition site and the TpG recognition site may be located within 40, 35, 30, 25, 20, 10, 9, 8, 7, 6, 5, 4, 3, or 2 bases from the 3' end of the methylation primer and unmethylation primer, or at the 3' end thereof.

The unmethylation primer has a low Tm value compared to the methylation primer, so in order to supplement this, by adding a nucleotide to the 5' end of the unmethylation primer, the Tm of the methylation primer and unmethylation primer may be similarly designed. For example, the unmethylation primer may have more nucleotides than the methylation primer, by further comprising a nucleotide at the 5' end. For example, the unmethylation primer may further comprise 1 to 5, 1 to 4, 1 to 3, 1 to 2, or 1 nucleotide at the 5' end compared to the methylation primer. For example, the Tm difference between the methylation primer and the unmethylation primer may be 15°C or less, 14°C or less, 13°C or less, 12°C or less, 11°C or less, 10°C or less, 9°C or less, 8°C or less, 7°C or less, 6°C or less, 5°C or less, 4°C or less, 3°C or less, 2°C or less, or 1.5°C or less.

The primer set comprises a forward primer and a reverse primer, and when the methylation primer and the unmethylation primer are forward primers, the counter direction primer is a reverse primer, and when the methylation primer and the unmethylation primer are reverse primers, the counter direction primer is a forward primer. The counter direction primer is a common primer capable of binding to both the methylation nucleic acid to be determined and unmethylation nucleic acid to be determined. Accordingly, the counter direction primer (common primer) may not comprise a CpG binding stie, but when it is inevitable that a CpG binding site is comprised in the counter direction primer due to the structure of the nucleic acid to be determined, 5 or less, 4 or less, 3 or less, 2 or less, 1 to 5, 1 to 4, 1 to 3, 1 to 2, or as one example, 1 CpG binding site may be comprised at the 5' end region. However, at this time, it is preferable that the counter direction primer is designed to have a Tm similar to the methylation primer and unmethylation primer.

The methylation primer, the unmethylation primer and the counter direction primer may be a size comprising 15 to 40, 15 to 35, 15 to 30, 15 to 25, 18 to 40, 18 to 35, 18 to 30, 18 to 25, 20 to 40, 20 to 35, 20 to 30, or 20 to 25 bases.

The methylation primer, the unmethylation primer and the counter direction primer may bind to a nucleic acid to be determined converted by an agent which modifies a methylated nucleic acid to be determined and an unmethylated nucleic acid to be determined differently from each other, by comprising 1 or more, 2 or more, or 3 or more Non-CpG cytosines.

The Tm of the methylation primer, the unmethylation primer and the counter direction primer may be 50 to 80°C, 50 to 75°C, 50 to 70°C, 50 to 65°C, 55 to 80°C, 55 to 75°C, 55 to 70°C, 55 to 65°C, 60 to 80°C, 60 to 75°C, 60 to 70°C, or 60 to 65°C.

The composition according to an embodiment of the present application may comprise the methylation primer and the unmethylation primer at a concentration ratio of 100:1 to 1:100, 100:1 to 1:50, 100:1 to 1:20, 100:1 to 1:10, 100:1 to 1:5, 100:1 to 1:1, 100:1 to less than 1:1, 100:1 to 1.3:1, 100:1 to 1.5:1, 100:1 to 2:1, 100:1 to 2.5:1, 100:1 to 3:1, 100:1 to 3.5:1, 100:1 to 4:1, 50:1 to 1:100, 50:1 to 1:50, 50:1 to 1:20, 50:1 to 1:10, 50:1 to 1:5, 50:1 to 1:1, 50:1 to less than 1:1, 50: 1 to 1.3:1, 50: 1 to 1.5:1, 50: 1 to 2:1, 50: 1 to 2.5:1, 50: 1 to 3:1, 50: 1 to 3.5:1, 50: 1 to 4:1, 10:1 to 1:100, 10:1 to 1:50, 10:1 to 1:20, 10:1 to 1:10, 10:1 to 1:5, 10:1 to 1:1, 10:1 to less than 1:1, 10:1 to 1.3:1, 10:1 to 1.5:1, 10:1 to 2:1, 10:1 to 2.5:1, 10:1 to 3:1, 10:1 to 3.5:1, 10:1 to 4:1, 5:1 to 1:100, 5:1 to 1:50, 5:1 to 1:20, 5:1 to 1:10, 5:1 to 1:5, 5:1 to 1:1, 5:1 to less than 1:1, 5:1 to 1.3:1, 5:1 to 1.5:1, 5:1 to 2:1, 5:1 to 2.5:1, 5:1 to 3:1, 5:1 to 3.5:1, 5:1 to 4:1, 4.5:1 to 1:100, 4.5:1 to 1:50, 4.5:1 to 1:20, 4.5:1 to 1:10, 4.5:1 to 1:5, 4.5:1 to 1:1, 4.5:1 to less than 1:1, 4.5:1 to 1.3:1, 4.5:1 to 1.5:1, 4.5:1 to 2:1, 4.5:1 to 2.5:1, 4.5:1 to 3:1, 4.5:1 to 3.5:1, or 4.5:1 to 4:1.

For example, the concentration of the unmethylation primer, may be 100% or less, less than 100%, 99.99% or less, 99.95% or less, 99.9% or less, 99.5% or less, 99% or less, 98% or less, 97% or less, 96% or less, 95% or less, 90% or less, 85% or less, 80% or less, 75% or less, 70% or less, 65% or less, 60% or less, 55% or less, 50% or less, 40% or less, 30% or less, or 25% or less of the concentration of the methylation primer, and as one example, it may be 50% or less.

The composition and method according to an embodiment of the present application may accurately determine whether a nucleic acid to be determined is methylated without using a fluorescent probe detecting an amplified product. Accordingly, the composition according to an embodiment of the present application may not comprise a fluorescent probe. In addition, the method according to an embodiment of the present application may not use a fluorescent probe.

The nucleic acid to be determined of which methylation is determined by the composition and method according to an embodiment of the present application may be comprised in a biological sample. The biological sample may comprise at least one selected from the group consisting of blood, serum, tissue, cells, feces and urine.

The composition and method according to an embodiment of the present application may determine whether a nucleic acid to be determined is methylated with high sensitivity, even when the concentration of the methylated nucleic acid to be determined in the biological sample is very low.

For example, the concentration of the methylated nucleic acid to be determined of the biological sample may be 100 ng/µl or less, 90 ng/µl or less, 80 ng/µl or less, 70 ng/µl or less, 60 ng/µl or less, 50 ng/µl or less, 40 ng/µl or less, 30 ng/µl or less, 20 ng/µl or less, 15 ng/µl or less, 10 ng/µl or less, 9 ng/µl or less, 8 ng/µl or less, 7 ng/µl or less, 6 ng/µl or less, 5 ng/µl or less, 4 ng/µl or less, 3 ng/µl or less, 2 ng/µl or less, 1.5 ng/µl or less, 1 ng/µl or less, 0.5 ng/µl or less, 0.4 ng/µl or less, 0.3 ng/µl or less, 0.2 ng/µl or less, 0.1 ng/µl or less, 0.05 ng/µl or less, 0.04 ng/µl or less, 0.03 ng/µl or less, 0.02 ng/µl or less, 0.01 ng/µl or less, 0.009 ng/µl or less, 0.008 ng/µl or less, 0.007 ng/µl or less, 0.006 ng/µl or less, or 0.005 ng/µl or less.

For example, the biological sample comprise a methylated nucleic acid to be determined and an unmethylated nucleic acid to be determined, and the concentration of the methylated nucleic acid to be determined may be 75% or less, 70% or less, 60% or less, 50% or less, 40% or less, 30% or less, 25% or less, 20% or less, less than 19%, 18% or less, 15% or less, 10% or less, 5% or less, 4.5% or less, 4% or less, 3.5% or less, 3% or less, 2.5% or less, 2% or less, 1.5% or less, 1.4% or less, 1.3% or less, 1.2% or less, or 1.1% or less of the concentration of the unmethylated nucleic acid to be determined.

For example, the biological sample comprises a methylated nucleic acid to be determined and an unmethylated nucleic acid to be determined, and it may comprise the methylated nucleic acid to be determined of 100% or less, less than 100%, 99% or less, 95% or less, 90% or less, 85% or less, 80% or less, 75% or less, 70% or less, 65% or less, 60% or less, 55% or less, 50% or less, 45% or less, 40% or less, 35% or less, 30% or less, 25% or less, 20% or less, 15% or less, 10% or less, less than 10%, 5% or less, less than 5%, 4.5% or less, 4% or less, 3.5% or less, 3% or less, 2.5% or less, 2% or less, 1.5% or less, 1.4% or less, 1.3% or less, 1.2% or less, 1.1% or less, or 1% or less, based on 100% of the total strands of the methylated nucleic acid to be determined and the unmethylated nucleic acid to be determined.

For example, the biological sample may comprise the methylated nucleic acid to be determined of 20,000 strands or less, 15,000 strands or less, 10,000 strands or less, 5,000 strands or less, 4,000 strands or less, 3,000 strands or less, 2,000 strands or less, 1,000 strands or less, 500 strands or less, 400 strands or less, 300 strands or less, 200 strands or less, 150 strands or less, 100 strands or less, 90 strands or less, 80 strands or less, 70 strands or less, 60 strands or less, 50 strands or less, 40 strands or less, 30 strands or less, 20 strands or less, 10 strands or less, 9 strands or less, 8 strands or less, 7 strands or less, 6 strands or less, 5 strands or less, 4 strands or less, or 3 strands or less.

The method for determining whether a nucleic acid to be determined is methylated according to an embodiment of the present application may further comprise a step of quantifying methylation level of the nucleic acid to be determined. The step of quantifying may be quantifying methylation level of the biological sample by comparing AUC (Area under the curve) of a melting curve of the biological sample, a sample in which the nucleic acid to be determined is 100% methylated, and a sample in which the nucleic acid to be determined is 100% unmethylated.

Specifically, the step of quantifying may comprise a step of obtaining a melt curve of the biological sample;
a step of obtaining a normalized melt curve of a biological sample in which the methylation ratio of the nucleic acid to be determined is identified; and a step of quantifying the methylation level of the biological sample, by comparing the melt curve of the biological sample and the normalized melt curve. The melt curve of the biological sample may be a normalized melt curve. The melt curve of the biological sample may be obtained through HRM analysis. The methylation ratio of the nucleic acid to be determined may be obtained at a content ratio of the methylated nucleic acid to be determined and unmethylated nucleic acid to be determined.

**In** other embodiment of the present application, the nucleic acid to be determined may comprise a biomarker for diagnosing cancer. Specifically, the nucleic acid to be determined may comprise a biomarker for diagnosing cancer, for example, a marker which is specifically methylated or unmethylated in cancer patients, and the target site of the nucleic acid to be determined may be a location of a biomarker for diagnosing cancer. **In** this case, it may be used for diagnosing cancer, by determining whether a nucleic acid to be determined is methylated with the composition or method according to an embodiment of the present application.

Accordingly, other embodiment of the present application relates to a composition for diagnosing cancer or a kit for diagnosing cancer, comprising the composition for determining whether a nucleic acid to be determined is methylated according to an embodiment of the present application.

**In** addition, other embodiment of the present application relates to a method for diagnosing cancer or a method for providing information for diagnosing cancer, comprising a step of determining whether a nucleic acid to be determined is methylated, by the method for determining whether a nucleic acid to be determined is methylated according to an embodiment of the present application. The method for diagnosing cancer or method for providing information for diagnosing cancer may further comprise a step of comparing the methylation level of the nucleic acid to be determined with the methylation level of the control group.

The control group means a nucleic acid isolated from a biological sample of which origin is already known, and all samples derived from a subject without liver cancer (normal control group) or a subject with liver cancer may be used.

For example, in case of a sample derived from an object without liver cancer (normal control group) is used as a control group, when the methylation level of the nucleic acid to be determined is higher than the methylation level of the nucleic acid isolated from the control group, or the nucleic acid to be determined with a higher methylation level than the control group is comprised in a biological sample, it may determine, diagnose, or provide information for diagnosis that an organism (for example, vertebrate, mammal, rodent, goat, deer, pig, bird, chicken, turkey, cow, horse, sheep, fish, primate, as one example, human) from which the nucleic acid to be determined or the biological sample is derived has cancer.

For example, in case of a sample derived from a subject with liver cancer is used as a control group, when the methylation level of the nucleic acid to be determined is similar to the methylation level of the nucleic acid isolated from the control group, or the nucleic acid to be determined with a similar methylation level to the control group is comprised in a biological sample, it may determine, diagnose, or provide information for diagnosis that an organism (for example, vertebrate, mammal, rodent, goat, deer, pig, bird, chicken, turkey, cow, horse, sheep, fish, primate, as one example, human) from which the nucleic acid to be determined or the biological sample is derived has cancer.

The cancer may include at least one selected from the group consisting of liver cancer, large intestine cancer, esophageal cancer, stomach cancer, rectal cancer, colorectal cancer, oral cancer, pharyngeal cancer, laryngeal cancer, lung cancer, non-small cell lung cancer, colon cancer, breast cancer, cervical cancer, endometrial cancer, ovarian cancer, prostate cancer, testis cancer, bladder cancer, kidney cancer, liver cancer, pancreatic cancer, biliary tract cancer, bone cancer, connective tissue cancer, skin cancer, melanoma, brain cancer, head and neck cancer, thyroid cancer, leukemia, Hodgkin disease, lymphoma, urinary tract cancer, and multiple myeloma blood cancer.

The liver cancer may be at least one selected from the group consisting of hepatocellular carcinoma, hepatoma, cholangiocarcinoma, intrahepatic cholangiocarcinoma, hepatoblastoma, hepatocarcinoma, hepatic hemangiosarcoma or metastatic liver cancer, vaguely nodular type HCC and hypovascular liver cancer.

Other embodiment of the present application relates to a method for treating cancer, comprising a step of treating a subject confirmed to have cancer by the composition for diagnosing cancer, the kit for diagnosing cancer, the method for diagnosing cancer, or the method for providing information for diagnosing cancer according to an embodiment of the present application. The step of treating may comprise a step of performing administration of an effective amount of a therapeutic agent to the subject, chemotherapy, hormone therapy, radiation therapy, a surgical operation, or a combination thereof.

The therapeutic agent may comprise for example, Afatinib, AK105, Anlotinib, Apatinib, Atezolizumab, Avelumab, axitinib, Bevacizumab, bosutinib, BSC, Cabozantinib, Cabozantinib-S-Malate, Camrelizumab, canertinib, carboplatin, capecitabine, celecoxib, CC-122, CF102, crizotinib, dasatinib, docetaxel, Donafenib, Dovitinib, doxorubicin, Durvalumab, EKB-569, entrectinib, epirubicin, erlotinib, etoposide, everollmus, FGF401, FOLFOX 4, fostamatinib, Galunisertib, gefitinib, gemcitabine, IBI305, ibrutinib, imatinib, INC280, Infigratinib, Ipilimumab, irinotecan, lapatinib, leflunomide, Lenvatinib, LY2875358, Mesylate, mitomycin c, MSC2156119J, neratinib, nilotinib, Nintedanib, Nivolumab, oxaliplatin, Palbociclib, Panobinostat, pazopanib, PDR001, Pembrolizumab, Pemigatinib, Pexavec, Phosphate, Ramucirumab, Regorafenib, ruxolitinib, semaxinib, selumetinib, SGO-110, SHR-1210, Sintilimab, Sorafenib, SU6656, sunitinib, sintilimab, spartalizumab, sutent, TACE, Tasquinimod, Temozolomide, Temsirolimus, Tislelizumab, Tivantinib, Tosylate, toripalimab, Tremelimumab, vandetanib, vatalanib, XL888, Y90, a pharmaceutically acceptable salt thereof, or a combination thereof.

### [ADVANTAGEOUS EFFECTS]

An embodiment of the present application can construct a design of a new experimental verification method by supplementing disadvantages of conventional qMSP, MethyLight and MS-HRM analysis methods, and can detect a DNA methylation marker through liquid biopsy, and has higher sensitivity than the conventional MS-HRM analysis method and quantification of the methylation level is possible.

An embodiment of the present application can conduct PCR at a more sensitive level for a target present in a very small amount such as circulating tumor DNA present in blood, and can design an experiment by adjusting the degree of binding of a methylation primer and an unmethylation primer to a template according to the use depending on the annealing temperature and the concentration ratio of the primer.

### [BRIEF DESCRIPTION OF THE DRAWINGS]

FIG. 1a to FIG. 1c are drawings that show the experiment results using MIP (methylation independent primer) for conventional MS-HRM analysis.
FIG. 2a to FIG. 2c are drawings that show the results of attempting improvement by comprising CpG at the 5'-end of a primer in order to inhibit unmethylation biased amplification occurring during PCR amplification using MIP.
FIG. 3a and FIG. 3b are drawings that confirm a phenomenon that interferes with amplification of a marker when a fluorescent probe used for Methylite analysis and the like is used.
FIG. 4a to FIG. 4c are drawings that show the results of performing MS-HRM analysis by mixing a methylation primer and an unmethylation primer.
FIG. 5a is a drawing that shows melting peak distribution of each sample according to the methylation ratio of a sample.
FIG. 5b is a drawing that shows the result of HRM analysis of each sample according to the methylation ratio of a sample by melting curve analysis.
FIG. 6a is a drawing that confirms whether quantitative amplification occurs when a methylation primer and an unmethylation primer are mixed and used in one test tube.
FIG. 6b is a drawing that shows the result of confirming accuracy of measurement of the methylation ratio using an mAUC (melting curve area under curve) representing the methylation ratio.
FIG. 6c is a drawing that confirms the method for determining methylation status according to an embodiment of the present application can distinguish between a methylated DNA concentration 1% level (3 strands of methylated DNA) and 0% concentration, and can detect 1% of methylated DNA in a sample in which unmethylated DNA is present at a concentration of 99%.
FIG. 7 is a drawing that confirms whether biased amplification of methylated DNA according to the concentration ratio of a methylation primer and an unmethylation primer occurs.
FIG. 8a and FIG. 8b are drawings that show the preliminary clinical results of diagnosing cancer by applying the method for determining methylation status according to an example of the present invention to actual blood.

### [MODE FOR INVENTION]

Hereinafter, the present application will be described in more detail by the following examples. However, these examples are intended to illustrate the present invention only, and the scope of the present application is not limited by these examples.

### Comparative Example 1. Limitation of conventional MS-HRM analysis method

As an example of a nucleic acid to be determined, a nucleic acid having the sequence of SEQ ID NO: 1 was used to confirm a limitation of the conventional MS-HRM analysis method. The nucleic acid sequence of SEQ ID NO: 1 is a liver cancer biomarker specifically methylated in a hepatoma cell. DNA isolated from the hepatoma cell may comprise the methylated nucleic acid of SEQ ID NO: 1, and DNA isolated from other cell may comprise the unmethylated nucleic acid of SEQ ID NO:1. Various samples comprising the nucleic acid of SEQ ID NO: 1 and a control sample were prepared as Table 1 and used for an experiment. Samples of Sample number 2 to 4 were purchased from Qiagen and used, and Sample number 2 was that all cytosine were converted into thymine by treating gDNA of Sample number 3 with sulfurous acid using EpiTect Bisulfite Kit, and Sample number 4 was that cytosine other than CpG cytosine were converted into thymine by treating gDNA of Sample number 3 with sulfurous acid-conversion using EpiTect Bisulfite Kit, after 100% methylated using SssI methylase. Samples of Sample number 5 to 7 were treated with sulfurous acid before PCR reaction, and unmethylated cytosine were converted into thymine.

**[Table 1]**

| Sample number | Sample name | Description for Sample | Methylated or not |
|---|---|---|---|
| 1 | NTC | non-template control | - |
| 2 | EpiTect Unmet. | 100% unmethylated sample | X |
| 3 | EpiTect gDNA | Sulfurous acid non-treated sample | - |
| 4 | EpiTect Met. | 100% methylated sample | O |
| 5 | HEK293T | Kidney cell-derived sample | X |
| 6 | Huh-1 | Hepatoma cell-derived sample | O |
| 7 | BS PBMC (NC114) | Blood cell-derived sample | X |

The conventional MS-HRM analysis uses a methylation independent primer (MIP) not comprising CpG and TpG recognition sites, in order to non-selectively amplify a methylation nucleic acid and an unmethylation nucleic acid treated with sulfurous acid. The sequences of the used methylation independent primers are as follows:
- Forward: gTtgTatTatTtgTTaggggTtgT (SEQ ID NO: 7)
- Reverse: ccacaAAAcctccaAAcaAtAA (SEQ ID NO: 8)

MS-HRM Master Mix was prepared by mixing in the composition of Table 2. The dose described in Table 2 is an amount required for one sample, and when several samples are tested, Master Mix at a level of the number of samples + 2 was prepared and used (22 µL/rxn).

**[Table 2]**

| Component | Volume |
|---|---|
| AccuPower^{®} PCR Master Mix 2X (Bioneer) | 12.5 uL |
| EvaGreen^{®} Dye 20X (Biotium) | 1.25 µL |
| Forward primer (10 uM) | 1 µL |
| Reverse primer (10 uM) | 1 µL |
| Nuclease free water (NFW) | 6.25 µL |

The prepared Master Mix was aliquoted in each well of a 96-Well PCR plate (Hard-Shell^{®} PCR Plates, Biorad) by 22 µL each. Seven kinds of the samples according to Table 1 were prepared to have a concentration of 0.33 ng/µL, and were aliquoted in each well by 3 µL each so that the total DNA amount was to be 1 ng. The PCR reaction was performed using CFX96 Touch Real-Time PCR Detection System (BioRad) at 95°C for 5 minutes once, and was performed by repeating the cycle of denaturation temperature of 95°C for 20 seconds, heating and cooling temperature of 60°C for 30 seconds, and extension temperature of 72°C for 30 seconds 50 times. In addition, the fluorescence value of each cycle was confirmed through measurement of fluorescence before staring the extension temperature after the heating and cooling temperature for 30 seconds. After completing the PCR reaction, after going through a stable period at 72°C for 5 minutes, melting analysis which confirms the degree of binding of amplified products according to the increase of the temperature was performed, and HRM analysis was carried out by measuring the intensity of fluorescence at 10 second intervals while increasing by 0.2°C from 65°C to 95°C.

The fluorescence values for the PCR and HRM analysis were confirmed, and a melting curve or peak was confirmed to determine characteristics of each sample. In addition, after completing PCR, electrophoresis was performed and confirmed the size of the amplified products was accurately generated by the designed primer.

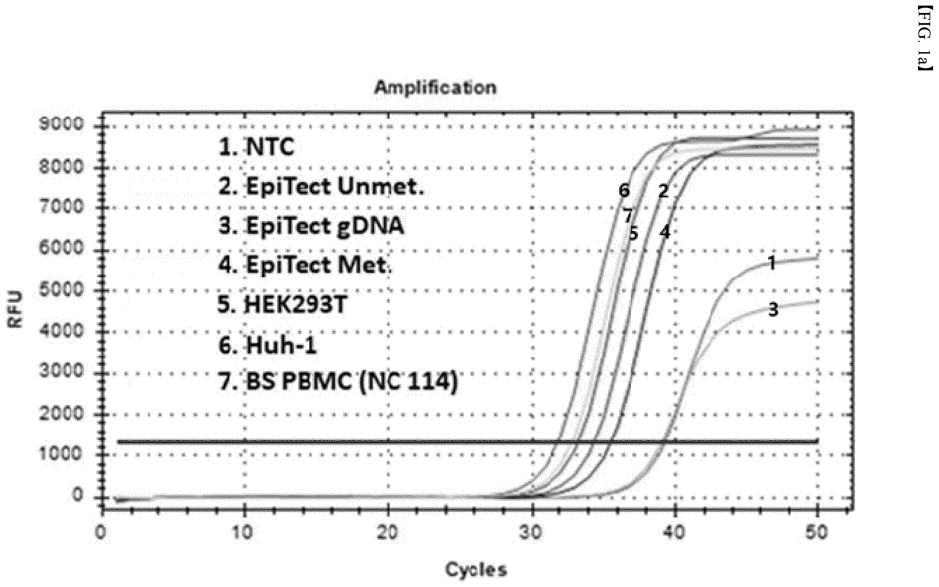

As shown in FIG. 1a, amplification of the sample in which the nucleic acid to be determined is 100% methylated (EpiTect Met; Sample number 4) and the nucleic acid to be determined is 100% unmethylated (EpiTect Unmet; Sample number 2) was shown almost similarly. In addition, they were amplified with similar efficiency in the hepatoma cell (Huh-1; Sample number 6), kidney cell (Hek293T; Sample number 5), and blood cell (PBMC; Sample number 7), and it was confirmed by an increase of a fluorescent RFU value of PCR that the methylated nucleic acid to be determined and unmethylated nucleic acid to be determined were both amplified. However, there was a problem in that non-specific amplification occurred by binding of the MIP even in the control group which was not treated with sulfurous acid (EpiTect gDNA; Sample number 3) and the control group not comprising nucleic acid to be determined (NTC; Sample number 1), which should not be amplified.

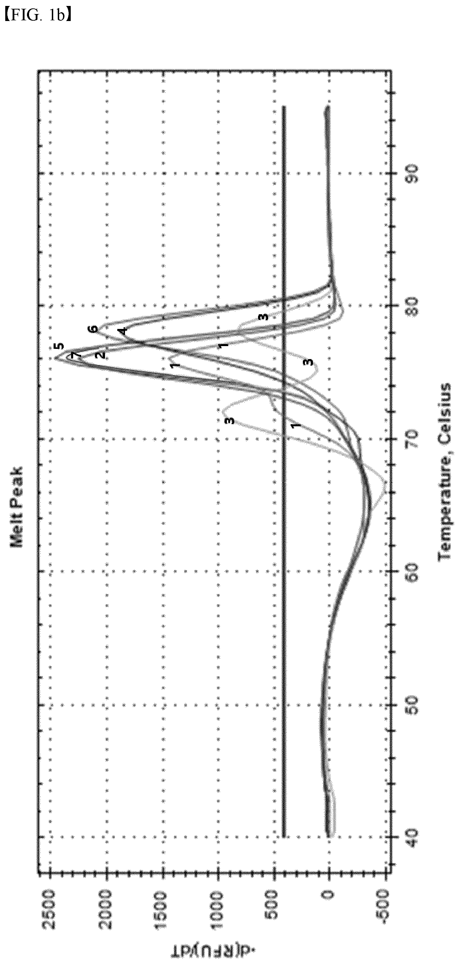

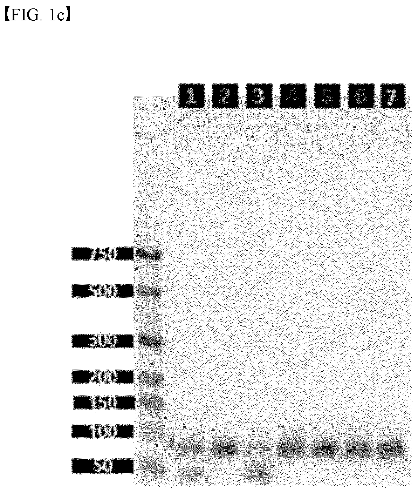

As shown in FIG. 1b, it could be confirmed that the amplified DNA was a methylation marker, an unmethylation marker, and a non-specific MIP amplified DNA through High Resolution Melting Curve (HRM) analysis. In addition, as electrophoresis result shown in FIG. 1c, it could be confirmed that a large number of non-specific amplified DNA was present.

Therefore, the conventional MS-HRM analysis method has a problem in that the genomic DNA sequence is simplified by treatment with sulfurous acid, and generation of non-specific amplified products frequently occurs due to simplicity of the MIP primer sequence only consisting of adenine, guanine and thymine, and amplification of a target nucleic acid is limited.

### Comparative Example 2. Limitation of MS-HRM analysis method using primer in which CpG recognition site is added in order to prevent biased amplification of unmethylated nucleic acid

In order to inhibit unmethylation biased amplification occurring during PCDR amplification using a methylation independent primer, a CpG sequence was comprised at the 5'-end of the primer to improve. The MIP primer used in Example 1 was changed to the following primers, and PCR was performed for the samples of Sample numbers 2, 3, 4 and 6 of Table 1 by the same method, and HRM analysis and electrophoresis were performed. The sequences of the used primers were as follows:
- Forward: TTtCGgTtgTatTatTtgTTaggggT (SEQ ID NO: 9)
- Reverse: AACGccccacaAAAcctccaAA (SEQ ID NO: 10)

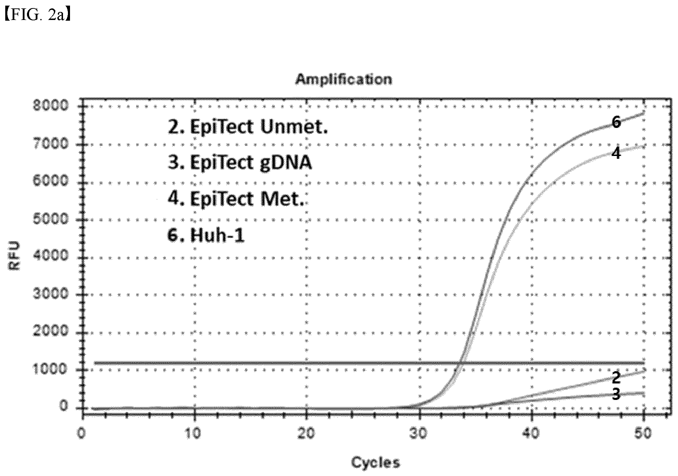

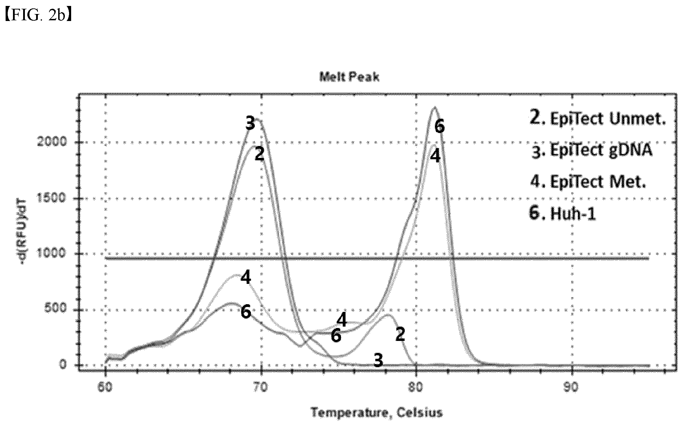

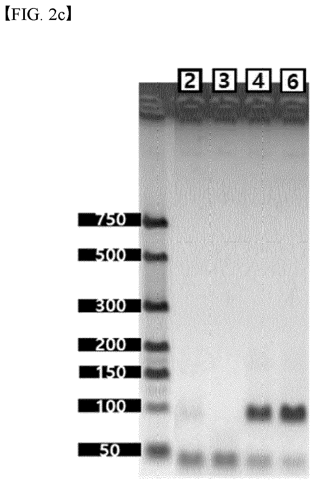

As shown in FIG. 2a, as a result of biasing so that methylated DNA could be amplified more favorably by adding CpG to the 5' end of MIP, it was confirmed that the 100% methylated sample (EpiTect Met; Sample number 4) and hepatoma cell DNA (Huh-1; Sample number 6) were effectively amplified, and the unmethylated DNA sample (EpiTect Unmet; Sample number 2) and control group not treated with sulfurous acid (Epitect gDNA; Sample number 3) were not amplified. However, as shown in the HRM analysis result of FIG. 2b and the electrophoresis analysis result of FIG. 2c, non-specific amplification occurred in the unmethylated sample. Therefore, there was a problem in that non-specific amplification very highly occurred when the unmethylated sample was analyzed under a condition in which bias was increased by methylation.

### Comparative Example 3. Limitation of MS-HRM analysis method using fluorescent probe

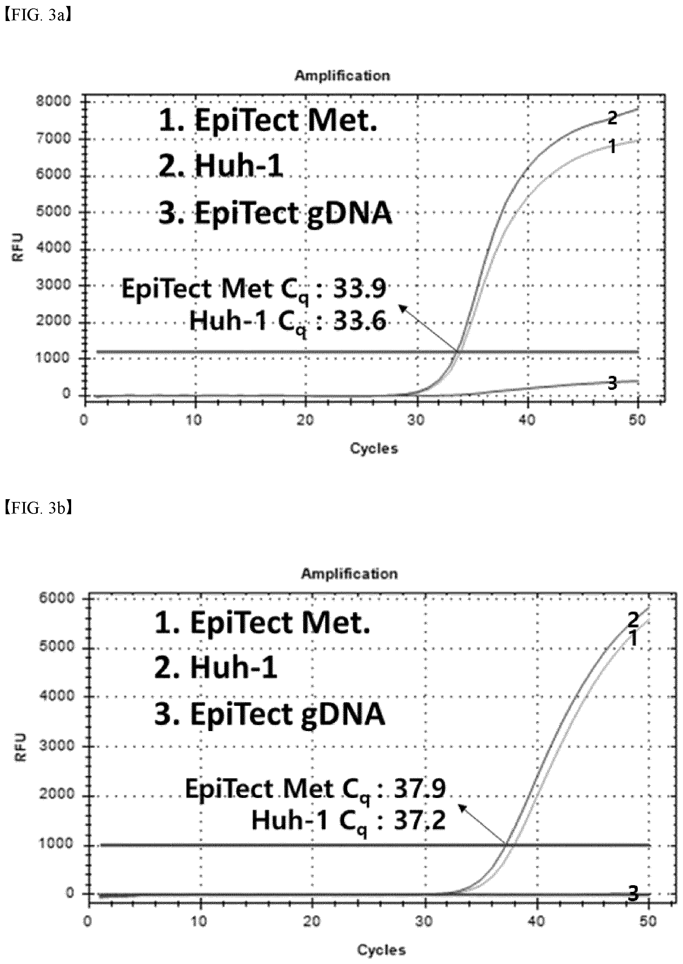

While performing as Comparative Example 1, PCR amplification efficiency was confirmed when using a fluorescent probe used for Methylite analysis, and the like. As Comparative Example 1, the result of performing MS-HRM for the liver cancer sample (Huh-1; Sample number 2) and 100% methylated sample (EpiTect Met; Sample number 1) was shown in FIG. 3a, and the result of performing in the same way by replacing 10 µL of NFW in the composition of Table 2 with a fluorescent probe (ggTTCGTTaCGTtgTTTt; SEQ ID NO: 11) was shown in FIG. 3b. In each experiment, as a control group, a sulfurous acid-untreated sample (EpiTect gDNA; Sample number 3) was used.

FIG. 3a is the result of measuring the amount of amplified DNA when a fluorescent probe which specifically binds to a methylated nucleic acid to be determined is not added with a PCR Cq value, and FIG. 3b is the result of performing by adding the fluorescent probe. As shown in FIG. 3b, the Cq value was increased about 4 due to the fluorescent probe, so there was a problem of decreasing the amplification efficiency of 10 to 20 times.

### Example 1. Manufacturing of methylation primer and unmethylation primer

A primer for determining whether a nucleic acid having the sequence of SEQ ID NO: 1 is methylated, as an example of the nucleic acid to be determined, was manufactured. The nucleic acid sequence of SEQ ID NO: 1 was shown in Table 3, and the target site was indicated in italics, and the primer binding site was indicated as underlined. In the sequence of SEQ ID NO: 1, CpG and Non-CpG cytosine were written in uppercase letters, and other bases were written in lowercase letters. In the sequence of SEQ ID NO: 1, the CpG sequence was indicated in bold.

The nucleic acid sequence after the methylated nucleic acid sequence of SEQ ID NO: 1 was treated with sulfurous acid was represented as SEQ ID NO: 2, and the nucleic acid sequence after the unmethylated nucleic acid sequence of SEQ ID NO: 1 was treated with sulfurous acid was represented as SEQ ID NO: 3. In SEQ ID NO: 2 and SEQ ID NO: 3, bases in which a difference occurred due to treatment with sulfurous acid according to methylated state of CpG cytosine were indicated in bold.

Considering the sequence of the template to be converted after treatment with sulfurous acid, one of forward or reverse primers was to comprise a CpG or TpG sequence near the 3'-end so as to specifically bind to one of methylated template or unmethylated template. The methylated template remained without being converted from CpG cytosine into thymine after treatment with sulfurous acid, so the methylation primer was designed to comprise a CpG recognition site which recognizes a CpG sequence, and the unmethylation primer was designed to comprise a TpG recognition site which recognizes a TpG sequence. At this time, since a primer specifically binding to an unmethylated template has a low Tm compared to a primer specifically binding to a methylated template, to supplement this, a nucleotide may be added to the 5'-end. The primer in the opposite direction to the methylation primer and unmethylation primer was designed to a primer capable of binding regardless of the methylation state of the methylated strand and unmethylated strand. All primers were allowed to specifically bind to a sulfurous acid-converted DNA strand by comprising 2 or more Non-CpG cytosines.

The forward primer was designed to bind to the 147th to 171th nucleic acids in the nucleic acid sequence of SEQ ID NO: 2 or SEQ ID NO: 3, and the reverse primer was manufactured to bind to the 206th to 229th nucleic acids in the nucleic acid sequence of SEQ ID NO: 2 or SEQ ID NO: 3. The primer binding site of SEQ ID NO: 2 or SEQ ID NO: 3 was indicated as underlined.

2 kinds of the forward primers were manufactured that a methylation primer capable of binding to the 147th to 171th nucleic acids in the nucleic acid sequence of SEQ ID NO: 2, and an unmethylation primer capable of binding to the 206th to 229th nucleic acids in the nucleic acid sequence of SEQ ID NO: 3. At this time, the reverse primer is a common reverse primer capable of binding to both the sulfurous acid-treated methylated nucleic acid to be determined and the sulfurous acid-treated unmethylated nucleic acid to be determined.

The sequences of the manufactured primers were shown in Table 3. In the sequences of the methylation primer and unmethylation primer, sequences that differ depending on whether a nucleic acid to be determined is methylated were indicated as underlined.

**[Table 3]**

| Classification | Sequence (5'-> 3') | SEQ ID NO. |
|---|---|---|
| Nucleic acid to be determined | aggaaggagaCGgggtggCGCCaaggaaggaggagaaaaggCGgCCGag aaaaggaggagggCaaggggaagaggaagggCGagggaggagCCtgaggaga CtCGCCCGgCtCaaCCCCGaCGtCCGCGCCCCGgCCGCCt gttggCCatggCGggCCtgggCCtgggCtCCGCCGttCCCGtgtggCtg gCCGaggaCGaCCtCGgCtgCatCatCtgCCaggggCtgCtggaCtgg CCCGCCaCGCtgCCCtgCGgCCaCagCttCtgCCGCCaCtgCCtg gaggCCCtgtggggCGCCCGCGaCGCCCGCCGCtgggCCtgCC CCaCttgCCGCCagggCGCCGCGCagCagCCGCaCCtgCGgaa gaaCaCGCtaCtgCaggaCCtggCCGaCaagtaCCGCCGCGCCG CaCGCGagataCaggCGggCtCCGaCCCtgCCCaCtgCCCCtgCC CGggCtCCagttCCCtCtCCag | 1 |
| Sulfurous acid-treated methylated nucleic acid to be determined | aggaaggagaCGgggtggCGTTaaggaaggaggagaaaaggCGgTCGag aaaaggaggagggTaaggggaagaggaagggCGagggaggagTTTgaggaga TtCGTTCGgTtTaaTTTCGaCGtTCGCGTTTCGgTCGTTTgttg gTTatggCGggTTTgggtTTgggTtTCGTCGttTTCGtgtggTtggTC GaggaCGaTTtCGgTtgTatTatTtgTTaggggTtgTtggaTtggTTCG TTaCGTtgTTTtgCGgTTaTagTttTtgTCGTTaTtgTTTggaggTTT tgtggggCGTTCGCGaCGTTCGTCGTTgggTTtgTTTTaTttgTC GTTagggCGTCGCGTagTagTCGTaTTtgCGgaagaaTaCGTtaT | 2 |
| | tgTaggaTTtggTCGaTaagtaTCGTCGCGTCGTaCGCGagataTa ggCGggTtTCGaTTTtgTTTaTtgTTTTtgTTCGggTtTTagttTTTt TtTTag | |
| Sulfurous acid-treated unmethylated nucleic acid to be determined | aggaaggagaTGgggtggTGTTaaggaaggaggagaaaaggTGgTTGaga aaaggaggagggTaaggggaagaggaagggTGagggaggagTTtgaggagaTt TGTTTGgTtTaaTTTTGaTGtTTGTGTTTTGgTTGTTtgttggT TatggTGggTTTgggtTTgggTtTTGTTGttTTTGtgtggTtggTTGag gaTGaTTtTGgTtgTatTatTtgTTaggggTtgTtggaTtggTTTGTTaT GTtgTTTtgTGgTTaTagTttTtgTTGTTaTtgtTTggaggTTTtgtggg gTGTTTGTGaTGTTTGTTGTtgggTTtgTTTTaTttgTTGTTag ggTGTTGTGTagTagTTGTaTTtgTGgaagaaTaTGTtaTtgTagga TTtggTTGaTaagtaTTGTTGTGTTGTaTGTGagataTaggTGgg TtTTGaTTTtgTTTaTtgTTTTtgTTTGggTtTTagttTTTtTtTTag | 3 |
| Forward methylation primer | TTatggCGggTTtgggTTtgggTtT | 4 |
| Forward unmethylation primer | TTatggTGggTTtgggTTtgggTtT | 5 |
| Common reverse primer | caAcccctAAcaAatAatAcaAcC | 6 |

### Example 2. MS-HRM using methylation primer and unmethylation primer

MS-HRM analysis was performed using the primer manufactured in Example 1. MS-HRM Master Mix was prepared by mixing in the composition of Table 4. The dose described in Table 4 is an amount required for one sample, and when several samples are tested, Master Mix at a level of the number of samples + 2 was prepared and used (22 µL/rxn).

**[Table 4]**

| Component | Volume |
|---|---|
| AccuPower^{®} PCR Master Mix 2X (Bioneer) | 12.5 µL |
| EvaGreen^{®} Dye 20X (Biotium) | 1.25 µL |
| Methylation forward primer (10 uM) | 1 µL |
| Unmethylation forward primer (10nM) | 0.5 µL |
| Common reverse primer (10 uM) | 1 µL |
| Nuclease free water (NFW): | 5.75 µL |

Various samples comprising the nucleic acid of SEQ ID NO: 1 and a control sample were prepared as Table 5 and used for an experiment.

**[Table 5]**

| Sample number | Sample name | Description for Sample | Methylated or not |
|---|---|---|---|
| 1 | NTC | non-template control | - |
| 2 | EpiTect gDNA | Sulfurous acid non-treated sample | - |
| 3 | EpiTect Unmet. DNA | 100% unmethylated sample | X |
| 4 | Healthy PBMC | Normal blood cell-derived sample | X |
| 5 | EpiTect Met. | 100% methylated sample | O |
| 6 | Huh-1 | Hepatoma cell-derived sample | O |

The prepared Master Mix was aliquoted in each well of a 96-Well PCR plate (Hard-Shell^{®} PCR Plates, Biorad) by 22 µL each. Six kinds of the samples according to Table 5 were prepared to have a concentration of 0.33 ng/µL, and were aliquoted in each well by 3 µL each so that the total DNA amount was to be 1 ng.

The PCR reaction was performed using CFX96 Touch Real-Time PCR Detection System (BioRad) at 95°C for 5 minutes once, and was performed by repeating the cycle of denaturation temperature of 95°C for 20 seconds, heating and cooling temperature of 60°C for 30 seconds, and extension temperature of 72°C for 30 seconds 50 times. In addition, the fluorescence value of each cycle was confirmed through measurement of fluorescence before staring the extension temperature after the heating and cooling temperature for 30 seconds. After completing the PCR reaction, after going through a stable period at 72°C for 5 minutes, melting analysis which confirms the degree of binding of amplified products according to the increase of the temperature was performed, and HRM analysis was carried out by measuring the intensity of fluorescence at 10 second intervals while increasing by 0.2°C from 65°C to 95°C.

The fluorescence values for the PCR and HRM analysis were confirmed, and a melting curve or peak was confirmed to determine characteristics of each sample. In addition, after completing PCR, electrophoresis was performed and confirmed that the size of the amplified products was accurately generated by the designed primer.

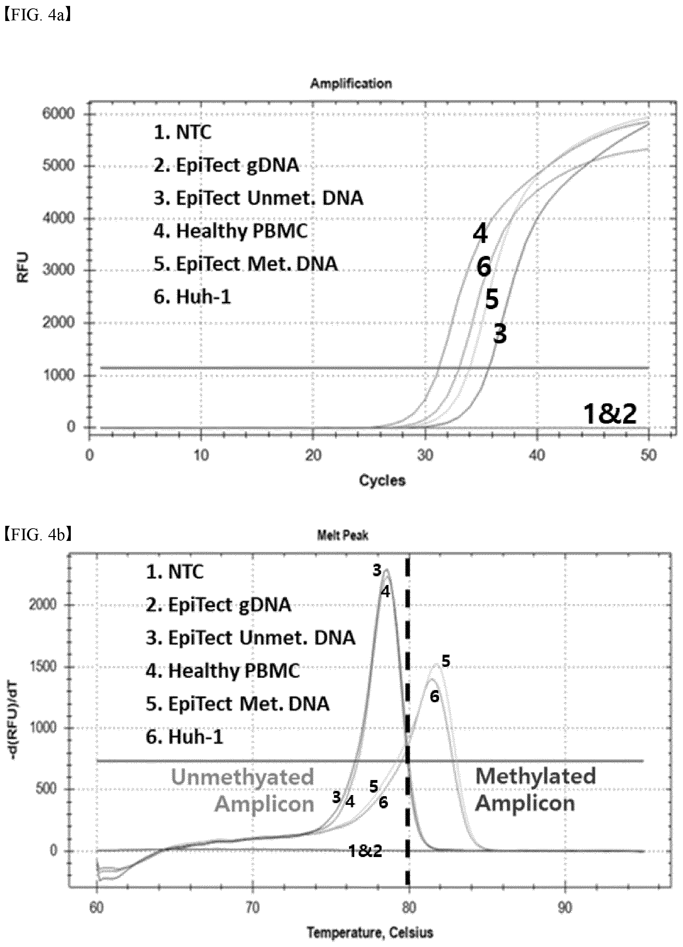

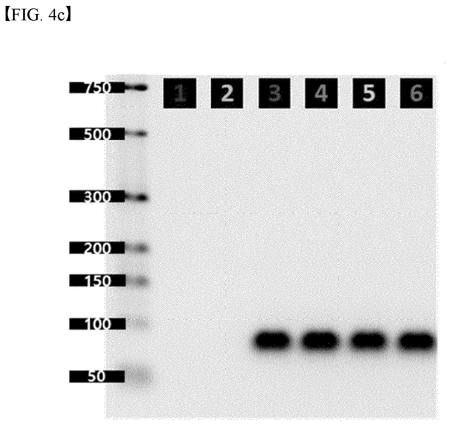

The methylation primer and unmethylation primer were simultaneously added to one sample, and a nucleic acid to be determined was amplified under a condition in which methylated DNA and unmethylated DNA could be amplified together. As shown in FIG. 4a, amplification occurred equally well in the sulfurous acid-treated methylated DNA (Epitect Met and Huh-1) and unmethylated DNA (EpiTect Unmet and PBMC), and amplification did not occur in the control sample (NTC and sulfurous acid-untreated EpiTect gDNA). It was confirmed that only specific amplification occurred in the HRM analysis result of FIG. 4b and the electrophoresis analysis result of FIG. 4c.

### Example 3. Limit of detection and quantification of methylation level

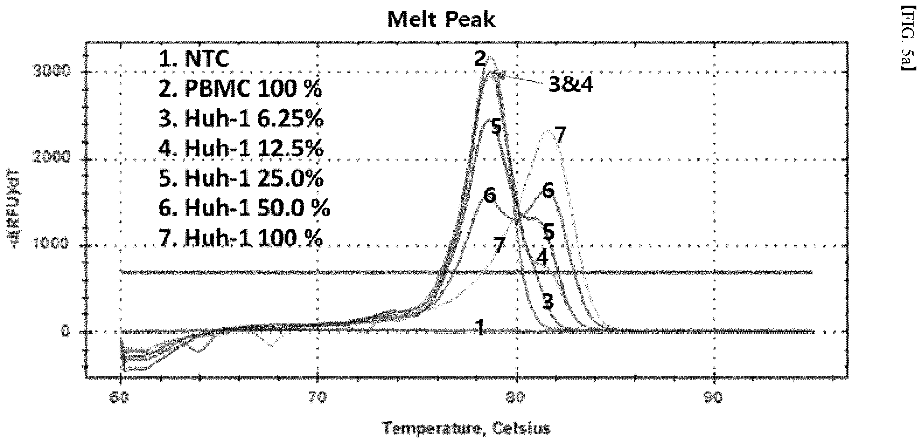

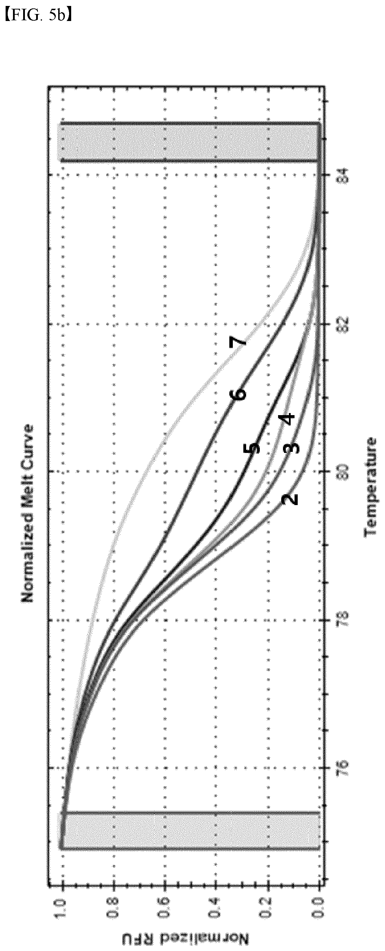

In order to confirm a minimal limit of detection of the methylation ratio of a sample, samples with a methylation ratio of 50%, 25%, 12.5%, or 6.25% were prepared by mixing PBMC (0.33 ng/µL) and Huh-1 (0.33 ng/µL) and used for an experiment. Each methylation ratio sample was aliquoted in each well by 3 µL, and PCR reaction and HRR analysis were performed by the same method as Example 2, and the result was shown in FIG. 5a and FIG. 5b.

FIG. 5a shows melting peak distribution of each sample by analyzing 1ng of DNA by the primer mixing MS-HRM method after mixing sulfurous acid-treated hepatoma cell DNA with sulfurous acid-treated blood cell DNA at a presented ratio. It can be seen that the size and ratio of the melting peak of the methylated DNA and the melting peak of the unmethylated DNA were gradually changed according to the ratio of the methylated Huh-1 DNA.

FIG. 5b is a diagram which shows the HRM analysis result by melting curve analysis. It shows that the slope of the melting curve gently increases according to the increase of hepatoma cell DNA, and it is located between the slope of the unmethylated blood cell and the slope of the 100% methylated hepatoma cell. Therefore, the area under the curve (AUC) of the melting curve can be calculated to quantitatively assess the methylation ratio. Specifically, the methylation level can be quantified by calculating the percentage obtained by dividing the difference between the AUC values of the melting curve for an unknown sample and the fully unmethylated control group by the difference between the AUC values of the melting curve for fully methylated control sample and the fully unmethylated control sample.

More specifically, in order to remove exponential background noise from the total fluorescence values according to the temperature, a temperature at which the MS-HRM fluorescence change rate is constant was selected in the section below and above the melting temperature (Tₘ) (each T_{L}, T_{R}), and a parameter of the exponential background noise was estimated from the fluorescence change rate (dF/dT) at each point. By calculating the exponential background noise value from the estimated parameter to remove it from the total fluorescence value by each temperature point, a fluorescence data function M(T) proportional to the amount of residual dsDNA is calculated. For M(T), min-max normalization is performed in the temperature section of [TL, TR] to calculate a residual dsDNA ratio function M1(T) according to the increase of temperature. In order to derive the methylation level, the area under the curve (AUC) value S of M1(T) in the [TL, TR] section was calculated from each sample, and then, the methylation level of the sample can be confirmed based on the S value.

### Example 4. Detection sensitivity

As ctDNA in blood is present at a very low concentration, in order to confirm whether the method for determining whether methylated according to an embodiment of the present application can be used for diagnosing cancer based on an actual blood test, a limit of detection was confirmed.

In order to confirm the minimal DNA strand which can be confirmed by the method for determining whether methylated according to an embodiment of the present application, PBMC and Huh-1 (50ng/3µL) samples were diluted with NFW to prepare Huh-1 samples at a concentration of 50, 30, 10, 5, 3, 1, 0.5, 0.3, 0.1, 0.05, or 0.01ng/3µL. The samples of each concentration were aliquoted in each well by 3 µL each, and PCR reaction and HRR analysis were performed by the same method as Example 2.

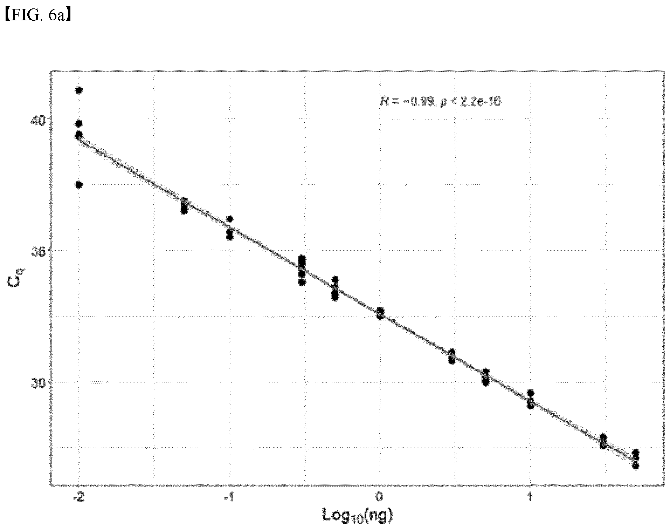

MS-HRM analysis with the methylation and unmethylation primer combination was performed using 5,000-fold different amounts of sulfurous acid-treated liver cancer cell DNA from 0.01ng to 50ng, and the amount of the amplified nucleic acid to be determined was analyzed by measurement of a PCR Cq value and was shown in FIG. 6a. As shown in FIG. 6a, as the result of amplifying while increasing DNA starting from a Cq value of about 38 when using 0.01ng (3 strands) of DNA to 50ng (15,200 strands), a decrease in the Cq value was shown in the relationship exactly same as the increase of the DNA amount. As a result, it could be seen that MS-HRM analysis using the combination of the methylation primer and unmethylation primer effectively amplified even 0.01ng of DNA and effectively amplified it in a broad region throughout 5,000 times.

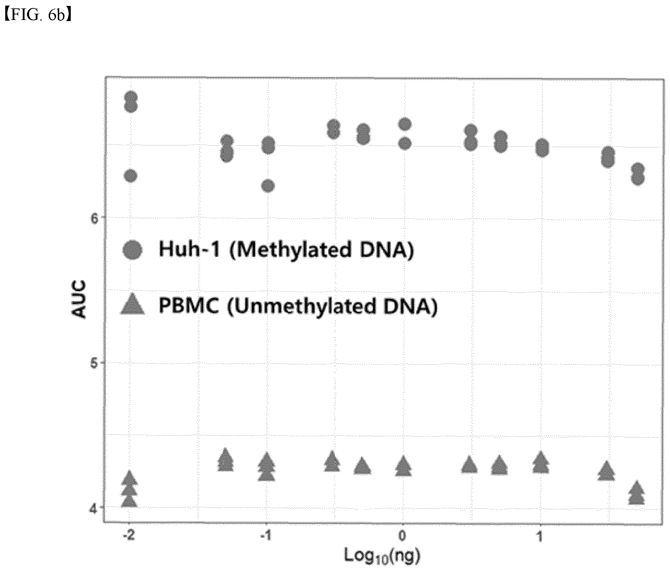

The result of analyzing whether accurate measurement of the methylation ratio is possible when analyzing various amounts of DNA using a mAUC (melting curve area under curve) showing a methylation ratio was shown in FIG. 6b. As shown in FIG. 6b, the difference in the AUC value was not large depending on the amount of each sample and was consistently confirmed, and the amplified product of each sample was a nucleic acid to be determined, and the methylation level of each sample could be determined. Therefore, even in the sample comprising at least 3 strands of the nucleic acid to be determined, methylated state of the nucleic acid to be determined could be determined.

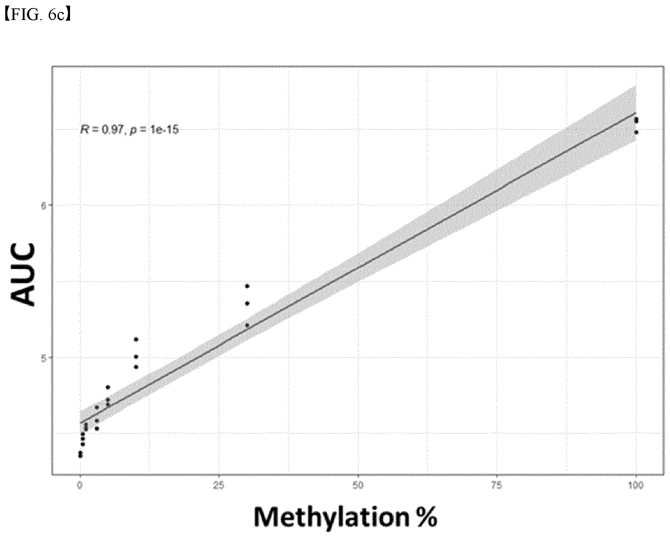

In addition, in order to confirm the minimal ratio in which the method for determining whether methylated according to an embodiment of the present application can determine target methylated DNA in an environment in which unmethylated DNA is present, samples of 0, 1, 2, 4, 8, 16, 32, 100 % were prepared by continuously diluting unmethylated DNA and methylated DNA and MS-HRM analysis was carried out, and the correlation of the change in the AUC value according to the ratio was confirmed and shown in FIG. 6c.

As shown in FIG. 6c, as the result of confirming the detectable minimal ratio under the condition in which unmethylated DNA is present, it could be confirmed that there was a difference in methylated DNA concentration of 1% (3 strands of methylated DNA) with 0% concentration, and a high R value could be confirmed even in the correlation analysis between the AUC value and the ratio of methylated DNA.

Thus, it was confirmed that the method for determining whether methylated according to an embodiment of the present application has a high detection sensitivity capable of detecting 1% of methylated DNA even in a sample in which unmethylated DNA of 99% is present.

### Example 6. Improvement of detection sensitivity by unmethylation primer

In order to confirm whether detection sensitivity is improved by an unmethylated primer, samples of Huh-1 6.25, 25.0, 50.0, or 100.0% were used. While performing as Example 5, the volume of the unmethylation forward primer was changed to 1, 0.75, 0.5, or 0.25 µL in the composition of Table 3, and accordingly, the NFW volume was changed to 5.25, 5.50, 5.75, or 6 µL.

Whether biased amplification of methylated DNA occurs according to the concentration ratio of the methylation primer and unmethylation primer was confirmed. PCR was performed under a condition in which the amount of the methylation primer was fixed to 0.4 µM, and the amount of the unmethylation primer was reduced to 0.4, 0.3, 0.2, 0.1µM.

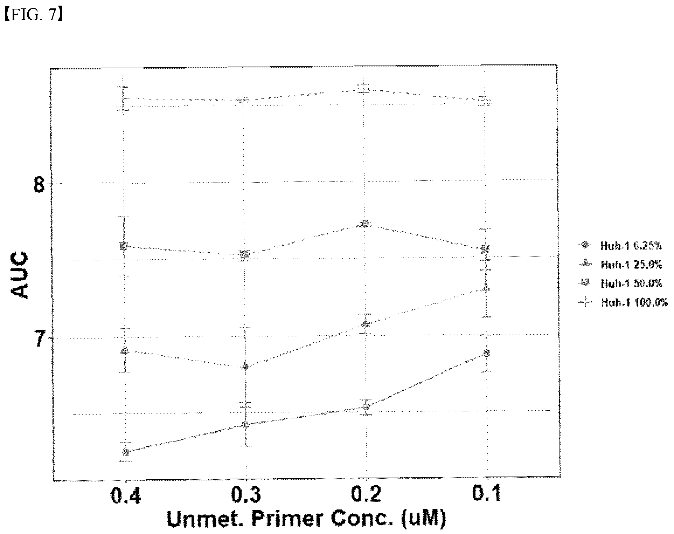

As shown in FIG. 7, when the DNA ratio of the methylated hepatoma cell was low as 25% or less, and the amount of the unmethylation primer was 50% or less of the methylation primer, the detection sensitivity of the methylated DNA was further increased.

Therefore, as the detection sensitivity increased by the use of the methylation primer and unmethylation primer in combination, whether methylation of methylated DNA, for example, cfDNA at a low concentration can be confirmed.

### Example 7. Cancer diagnosis of measuring methylation of cancer biomarker in blood

Preliminary clinical trials were performed to confirm whether determining methylated state of a nucleic acid to be determined is possible by applying MS-HRM analysis with combination of methylation primer and unmethylation primer to actual blood.

After extracting 10 mL of blood from healthy people and liver cancer patients, serum was separated using a centrifuge (1,900g, 15 minutes). According to the method suggested by the manufacturer, cfDNA was extracted from the separated serum using MagListo^{™} cfDNA Extraction Kit (Bioneer). For extracted cfDNA, sulfurous acid conversion was carried out for MS-HRM analysis using EZ DNA Methylation-Lightning Kits (Zymo Research) according to the method suggested by the manufacturer. For the sulfurous acid-converted cfDNA, MS-HRM analysis was performed through the next process.

MS-HRM Master Mix was prepared according to Table 3, and the prepared Master Mix was aliquoted in each well of a 96-Well PCR plate (Hard-Shell^{®} PCR Plates, Biorad) by 22 µL each, and PCR reaction and HRR analysis were performed by the same method as Example 2. The fluorescence value for the PCR and HRM analysis was confirmed, and the melting curve or peak was confirmed, and thereby, the methylation level of the healthy people and liver cancer patients was determined.

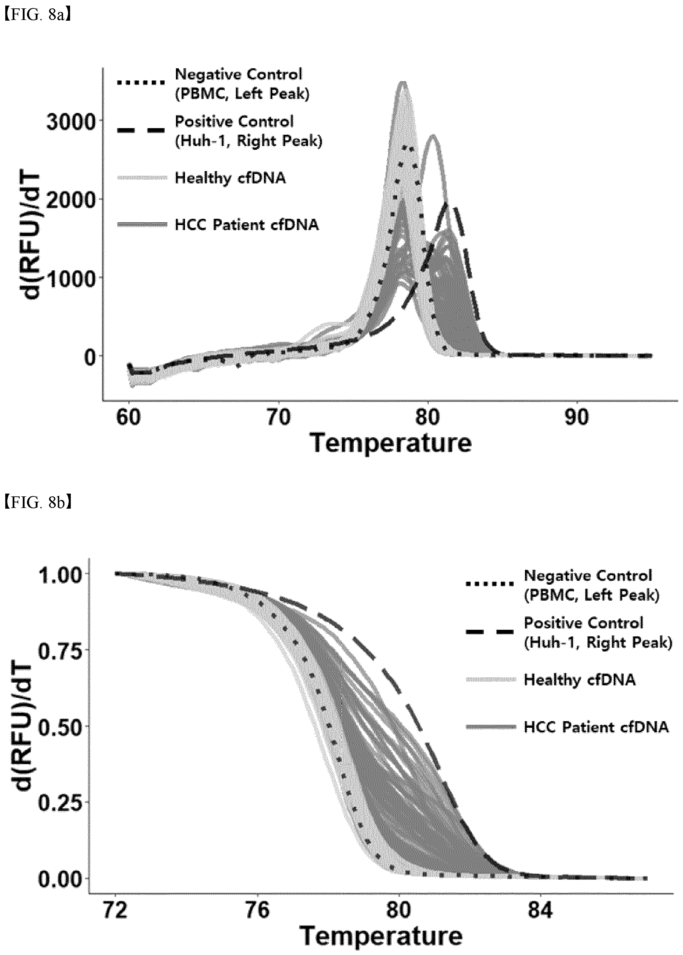

FIG. 8a is a drawing which shows the result of primer combination MS-HRM using about 1ng of cfDNA, after collecting 2ml of blood from 92 healthy people and 119 liver cancer patients to separate plasma, and separating cfDNA therefrom and treating with sulfurous acid. The analysis result of the healthy people (green) represents a melting peak at a low temperature where all are unmethylated, and the blood samples of the liver cancer patients (red) represent a melting peak at a high temperature.

FIG. 8b is a drawing that shows the experimental result represented by melting curve analysis for a nucleic acid to be determined of SEQ ID NO: 1 using combination MS-HRM method for blood of normal people and liver cancer patients. Normal people (green) is unmethylated, so the amplified DNA melts quickly and shows a rapid curve slope, while the liver cancer patient blood samples (red) comprise cancer DNA having a high melting point due to amplification of methylated cancer DNA, so it shows a relatively slowly melting curve slope. It can be confirmed that about 60% or more of liver cancer patient cfDNA comprise higher methylated DNA than cfDNA of the normal people, and thus, a trace amount of methylation marker DNA present in blood can be effectively detected by detecting a very small amount of nucleic acid such as cfDNA, for example, methylated cfDNA fragmented very small of about 100bp.

## Claims

1. A composition for determining whether a nucleic acid to be determined is methylated, comprising a primer set for amplifying a target site of the nucleic acid to be determined, wherein the primer set comprises
(1) a methylation forward primer, an unmethylation forward primer, and a common reverse primer; or
(2) a common forward primer, a methylation reverse primer, and an unmethylation reverse primer,
wherein the methylation forward primer and the methylation reverse primer comprise a CpG recognition site which recognizes a CpG sequence of the nucleic acid to be determined to be determined,
wherein the unmethylation forward primer and the unmethylation reverse primer comprise a TpG recognition site which recognizes a TpG sequence of the nucleic acid to be determined, and
wherein the TpG sequence is converted from the CpG sequence of the nucleic acid to be determined.

2. The composition according to claim 1, wherein the TpG sequence is converted by an agent which modifies a methylated nucleic acid to be determined and an unmethylated nucleic acid to be determined differently each other.

3. The composition according to claim 2, wherein the agent converts an unmethylated cytosine residue into thymine.

4. The composition according to claim 2, wherein the agent is at least one selected from the group consisting of sulfurous acid, bisulfite, hydrogen sulfite and disulfite.

5. The composition according to claim 1, wherein the CpG recognition site or the TpG recognition site is located within 40 bases from the 3' end of the methylation forward primer, the methylation reverse primer, the unmethylation forward primer, or the unmethylation reverse primer.

6. The composition according to claim 1, wherein the target site comprises a CpG sequence.

7. The composition according to claim 1, wherein the target site is 50 to 150 bp in size.

8. The composition according to claim 1, wherein the primer has a size of comprising 15 to 40 bases.

9. The composition according to claim 1, wherein the common reverse primer or the common forward primer comprises 5 or less of CpG or TpG recognition sites of the nucleic acid to be determined.

10. The composition according to claim 1, wherein the Tm difference of the methylation forward primer and unmethylation forward primer of (1); or the Tm difference of the methylation reverse primer and unmethylation reverse primer of (2) is 15°C or less.

11. The composition according to claim 1, wherein
the methylation forward primer and unmethylation forward primer of (1); or
the methylation reverse primer and unmethylation reverse primer of (2)
are comprised at a concentration ratio of 100:1 to 1:100.

12. The composition according to claim 1, wherein the nucleic acid to be determined is comprised in a biological sample.

13. The composition according to claim 12, wherein the biological sample comprises at least one selected from the group consisting of blood, serum, tissue, cells, feces and urine.

14. The composition according to claim 12, wherein the biological sample comprises a methylated nucleic acid to be determined and an unmethylated nucleic acid to be determined.

15. The composition according to claim 14, wherein the concentration of the methylated nucleic acid to be determined is 75% or less of the concentration of the unmethylated nucleic acid to be determined.

16. The composition according to claim 1, wherein the nucleic acid to be determined comprises a biomarker for diagnosing cancer.

17. The composition according to claim 16, wherein the cancer comprises at least one selected from the group consisting of liver cancer, large intestine cancer, esophageal cancer, stomach cancer, rectal cancer, colorectal cancer, oral cancer, pharyngeal cancer, laryngeal cancer, lung cancer, non-small cell lung cancer, colon cancer, breast cancer, cervical cancer, endometrial cancer, ovarian cancer, prostate cancer, testis cancer, bladder cancer, kidney cancer, liver cancer, pancreatic cancer, biliary tract cancer, bone cancer, connective tissue cancer, skin cancer, melanoma, brain cancer, head and neck cancer, thyroid cancer, leukemia, Hodgkin disease, lymphoma, urinary tract cancer, and multiple myeloma blood cancer.

18. The composition according to claim 1, wherein the target site is a biomarker located in a CpG island.

19. A method for determining whether a nucleic acid to be determined is methylated, comprising
a step of modifying a methylated nucleic acid to be determined and an unmethylated nucleic acid to be determined differently from each other, wherein the nucleic acid to be determined is comprised in a biological sample; and
a step of amplifying a target site by treating the biological sample with the composition according to any one of claims 1 to 18.

20. The method according to claim 19, wherein the modifying is performed by treating the biological sample with an agent which modifies a methylated nucleic acid to be determined and an unmethylated nucleic acid to be determined differently from each other.

21. The method according to claim 19, wherein the biological sample is at least one selected from the group consisting of blood, serum, tissue, cells, feces and urine.

22. The method according to claim 19, further comprising a step of quantifying methylation level of the nucleic acid to be determined.

23. The method according to claim 22, wherein the step of quantifying is quantifying the methylation level of the biological sample, by comparing AUC (Area under the curve) of a normalized melt curve of the biological sample, a sample in which the nucleic acid to be determined is 100% methylated, and a sample in which the nucleic acid to be determined is 100% unmethylated.

24. The method according to claim 19, further comprising
a step of obtaining a melt curve of the biological sample;
a step of obtaining a normalized melt curve of a biological sample in which the methylation ratio of the nucleic acid to be determined is identified; and
a step of quantifying methylation level of the biological sample, by comparing the melt curve of the biological sample and the normalized melt curve.

25. The method according to claim 19, wherein the target site comprises a biomarker located in a CpG island.

26. A method for providing information for diagnosing cancer, comprising
a step of modifying a methylated nucleic acid to be determined and an unmethylated nucleic acid to be determined differently from each other, in a nucleic acid to be determined comprised in a biological sample;
a step of amplifying by treating the biological sample with the composition according to any one of claims 1 to 18; and
a step of determining whether a nucleic acid to be determined is methylated,
wherein the nucleic acid to be determined comprises a biomarker for diagnosing cancer.

27. The method according to claim 26, wherein the biomarker nucleic acid for diagnosing cancer is methylated or unmethylated in a cancer patient.

28. The method according to claim 26, further comprising a step of comparing the methylation level of the nucleic acid to be determined with the methylation level of a control group.

29. The method according to claim 26, wherein the cancer comprises one or more selected from the group consisting of
liver cancer, large intestine cancer, esophageal cancer, stomach cancer, rectal cancer, oral cancer, pharyngeal cancer, laryngeal cancer, lung cancer, colon cancer, breast cancer, cervical cancer, endometrial cancer, ovarian cancer, prostate cancer, testis cancer, bladder cancer, kidney cancer, liver cancer, pancreatic cancer, bone cancer, connective tissue cancer, skin cancer, brain cancer, thyroid cancer, leukemia, Hodgkin disease, lymphoma, and multiple myeloma blood cancer.

30. The method according to claim 26, wherein the biomarker for diagnosing cancer is located in a CpG island.

31. A kit for determining whether a nucleic acid to be determined is methylated, comprising the composition according to any one of claims 1 to 18.

32. A composition for diagnosing cancer, comprising the composition according to any one of claims 1 to 18, wherein the nucleic acid to be determined comprises a cancer biomarker.

33. A kit for diagnosing cancer, comprising the composition according to claim 30.
